(19) Europäisches Patentamt
European Patent Office
Office européen des brevets

(11) Veröffentlichungsnummer : **0 002 500**
**B1**

(12) **EUROPÄISCHE PATENTSCHRIFT**

(45) Veröffentlichungstag der Patentschrift : **30.03.83**

(51) Int. Cl.³ : **C 07 D337/14**, C 07 D409/12, A 61 K 31/38, A 61 K 31/445

(21) Anmeldenummer : **78101593.8**

(22) Anmeldetag : **07.12.78**

(54) **Aminoalkylthiodibenzthiepine und -derivate, diese Verbindungen zur Verwendung als Arzneimittel, Verfahren und Zwischenprodukte zu ihrer Herstellung.**

(30) Priorität : **13.12.77 US 860082**

(43) Veröffentlichungstag der Anmeldung : **27.06.79 Patentblatt 79/13**

(45) Bekanntmachung des Hinweises auf die Patenterteilung : **30.03.83 Patentblatt 83/13**

(84) Benannte Vertragsstaaten : **BE CH DE FR GB IT NL**

(56) Entgegenhaltungen :
**CHEMICAL ABSTRACTS, Vol. 76, 1972, Nr. 14370b, Columbus, Ohio, USA, JILEK JIRI et al. « Neurotropic basic enol ethers of the dibenzo (b,f)-thiepin series », Seite 371, linke Spalte**

**Medicinal Chemistry, Third Ed., Part I, Burger A, New York, U.S.A. 1970, Seite 75, Part II Seiten 1410-1412 Pschyembel, Klin. Wörterbuch, 253 Aufl. Berlin, New York 1977, S. 996-997**

**Die Akte enthält technische Angaben, die nach dem Eingang der Anmeldung eingereicht wurden und die nicht in dieser Patentschrift enthalten sind.**

(73) Patentinhaber : **HOECHST AKTIENGESELLSCHAFT**
**Postfach 80 03 20**
**D-6230 Frankfurt/Main 80 (DE)**

(72) Erfinder : **Ong, Helen Hu**
**Bunker Hill Place 15**
**Whippany New Jersey (US)**
Erfinder : **Anderson, Vernon Brian**
**Sylvan Road 24**
**High Bridge New Jersey (US)**
Erfinder : **Profitt, James Arthur**
**Deanna Drive 34**
**Somerville New Jersey (US)**

**EP 0 002 500 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Aminoalkylthiodibenzthiepine und -derivate, diese Verbindungen zur Verwendung als Arzneimittel, Verfahren und Zwischenprodukte zu ihrer Herstellung

Die vorliegende Erfindung betrifft neue Aminoalkylthiodibenzthiepine, deren Derivate und physiologisch verträgliche Säureadditionssalze, sowie Human- und Veterinärpräparate, die als Wirkstoffe diese Verbindungen enthalten.

Es ist bereits bekannt, daß Amethoclothiepin der allgemeinen Formel

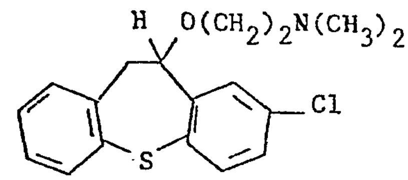

eine ZNS-sedierende Wirkung hat (M. Protvia, et al. Il Farmaco *XXI* 98 (1966)).

Das japanische Patent Nr. 47-28998 mit dem Titel « Verfahren zur Herstellung von trizyklischen Verbindungen mit einer enolischen Ätherverbindung » betrifft die Herstellung von Verbindungen der allgemeinen Formel

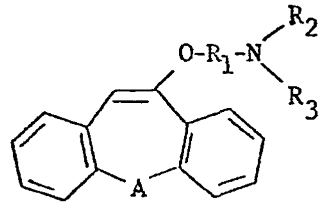

worin A eine Alkylimino-, Oxy-, Thio- oder Sulfonylgruppe und $R_1$ Alkylen bedeutet, $R_2$ und $R_3$ jeweils eine Alkylgruppe bedeuten oder entweder durch Alkylimin oder nicht durch Alkylimin zyklisch verbunden sein können.

Die Verbindungen der vorliegenden Erfindung unterscheiden sich jedoch beträchtlich von denen des Standes der Technik.

Gegenstand der vorliegenden Erfindung sind Verbindungen der Formel

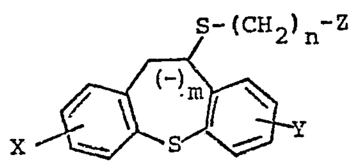

(I)

worin

X und Y gleich oder verschieden sein können und jeweils Wasserstoff, Halogen, $C_{1-6}$-Alkylsulfonyl bedeuten,

Z für

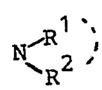

steht, wobei $R^1$ Wasserstoff, geradkettiges oder verzweigtes $C_1$-$C_6$-Alkyl, Cyano bedeutet, $R^2$ geradkettiges oder verzweigtes $C_{1-6}$-Alkyl bedeutet oder $R^1$ und $R^2$ zusammen mit dem Stickstoffatom, an das sie gebunden sind, den Morpholin-Ring oder ein N-substituiertes Piperazinyl, worin der N-Substituent $C_{1-6}$-Alkyl ist, bilden,

m für 0 oder 1 steht und

n eine Zahl von 2 bis 4 bedeutet,

sowie deren physiologisch verträgliche Säureadditionssalze.

Die vorliegende Erfindung betrifft ferner Verbindungen der obigen Formel I, worin X, Y, m und n die oben genannten Bedeutungen haben, Z für ein Halogenatom oder den Rest

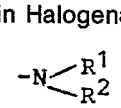

steht, wobei $R^1$ ein Phenoxycarbonyl oder $C_{1-6}$-Alkoxycarbonyl und $R^2$ geradkettiges oder verzweigtes $C_{1-6}$-Alkyl bedeuten, als geeignete Zwischenprodukte für die Herstellung der oben genannten Verbindungen der Formel I mit antidepressiven, analgetischen und anticonvulsiven Eigenschaften.

Geeignete Säuren zur Herstellung der pharmazeutisch verträglichen Säureadditionssalze gemäß der

Erfindung sind z. B. anorganische Säuren wie Salzsäure, Bromwasserstoffsäure, Schwefelsäure, Salpetersäure, Phosphorsäure und Perchlorsäure oder organische Säuren wie Weinsäure, Zitronensäure, Essigsäure, Bernsteinsäure, Maleinsäure, Fumarsäure und Oxalsäure.

Die Verbindungen der Erfindungen können nach einer der unten genannten Methoden hergestellt werden, wobei X, Y, Z, $R^1$, $R^2$, m und n, wenn nicht anders angegeben, die oben genannte Bedeutung haben.

a) Ein 10,11-Dihydro-10-hydroxy- oder -10-oxo-dibenz-[b,f]-thiepin der Formel

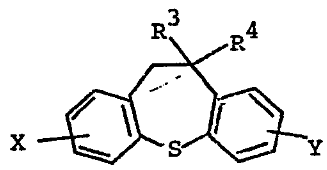

(II)

worin X und Y die in Formel I angegebene Bedeutung haben und $R^3$ und $R^4$ verschieden sind und Wasserstoff oder Hydroxy oder zusammen Sauerstoff bedeuten, wird mit Aminoalkylthiol der Formel

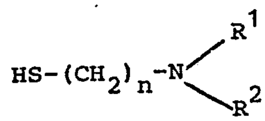

(III)

worin $R^1$ und $R^2$ gleich oder verschieden sind und geradkettiges oder verzweigtes $C_{1-6}$-Alkyl bedeuten, zu einer Verbindung der Formel

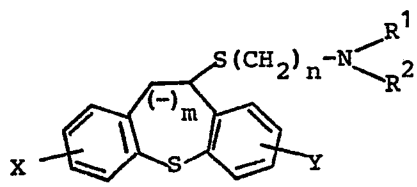

(Ia)

umgesetzt.

b) Eine gemäß a) hergestellte Verbindung, worin $R^1$ und $R^2$ Methyl bedeuten, wird mit Halogencyan wie z. B. Bromcyan in einem geeigneten Lösungsmittel und in Gegenwart eines Säurefängers bei einer Temperatur von Zimmertemperatur bis Rückflußtemperatur zu einem Gemisch einer Verbindung der Formel

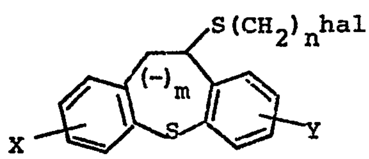

(Ib)

und einer Verbindung der Formel

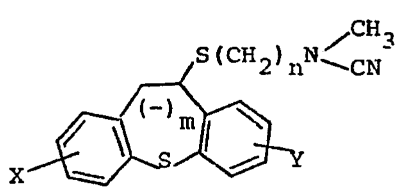

(Ic)

umgesetzt. Diese beiden Verbindungen können dann durch Säulenchromatographie getrennt werden.

c) Eine gemäß b) hergestellte Verbindung der Formel Ib wird in bekannter Weise mit einem geeignetem Amin zur entsprechenden Verbindung der Formel

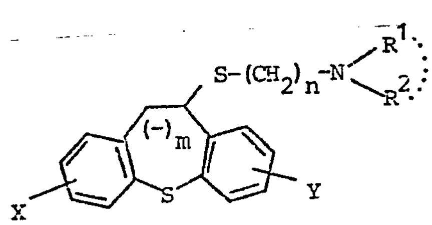

(Ia)

worin $R^1$ Wasserstoff, verzweigtes oder unverzweigtes $C_{1-6}$-Alkyl, $R^2$ geradkettiges oder verzweigtes $C_{1-6}$-Alkyl oder $R^1$ und $R^2$ zusammen mit dem Stickstoffatom, an das sie gebunden sind, N-substituiertes Piperazinyl oder den Morpholin-Ring bilden.

Vorzugsweise arbeitet man in Dimethylformamid als Lösungsmittel und in Gegenwart eines Säurefängers wie Natriumbicarbonat und eines Reaktionsinitiators wie Kaliumiodid, bei einer Temperatur von Zimmertemperatur bis Rückflußtemperatur des Reaktionsgemisches.

d) Eine gemäß a) hergestellte Verbindung wird mit Chlorameisensäureester wie z. B. Alkyl- oder Phenylchlorameisensäureester, bei einer Temperatur von 25-125 °C, in einem Lösungsmittel wie Methylenchlorid, Toluol oder Benzol zur entsprechenden Verbindung der Formel I, worin Z die Gruppe

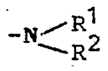

darstellt, worin $R^1$ $C_{1-6}$-Alkoxy oder Phenoxycarbonyl bedeutet, umgesetzt.

e) Eine gemäß d) hergestellte Verbindung wird mit einer Base wie Natrium- oder Kaliumhydroxyd in einem Lösungsmittel wie Wasser, Äthanol oder Äthylenglykol, bei einer Temperatur von Zimmertemperatur bis Rückflußtemperatur oder in einem saurem Milieu wie Bromwasserstoff in Eisessig, bei einer Temperatur von etwa Zimmertemperatur bis Rückflußtemperatur zur entsprechenden Verbindung der Formel I, worin $R^1$ Wasserstoff ist, umgesetzt.

f) Eine gemäß e) hergestellte Verbindung wird mit einem geradkettigen oder verzweigten $C_{1-6}$-Alkylhalogenid, unter für eine solche Reaktion üblichen Bedingungen zur entsprechenden Verbindung der Formel I, worin $R^1$ geradkettiges oder verzweigtes $C_{1-6}$-Alkyl, bedeutet, vorzugsweise in Gegenwart eines Lösungsmittels wie Dimethylformamid, eines Säurefängers wie Natriumbicarbonat und eines Reaktionsinitiators wie Kaliumiodid, bei Rückflußtemperatur des Lösungsmittels, umgesetzt.

Selbstverständlich hängen die speziellen Reaktionsbedingungen von den jeweils eingesetzten Substanzen ab.

Die Verbindungen der Erfindung eigenen sich zur Behandlung von Depressionen, was an ihrer Fähigkeit, eine Tetrabenazin-induzierte Depression bei Mäusen zu unterdrücken, ersichtlich wird [International Journal of Neuropharmacology *8,* 73 (1969)]. Dieser Test ist ein Standardtest zur Feststellung antidepressiver Eigenschaften.

Die Verbindungen eignen sich weiterhin als Analgetica aufgrund ihrer Fähigkeit, Schmerzen in Säugetieren zu lindern, was man mit dem Phenyl-p-Chinon-Strecktest an Mäusen, einem Standardtest für Analgetica, nachweisen kann [Proc. Soc. Exptl. Biol. Med., *95,* 729 (1957)].

Die Verbindungen der Erfindung eignen sich weiterhin als Anticonvulsiva, was mit der Methode nach L. A. Woodbury, und V. D. Davenport (Arch. Int. Pharmacodynam, Vol. *92* (1952), Seiten 97-107) nachgewiesen werden kann.

Die erfindungsgemäßen Verbindungen sind hinsichtlich der 3 oben genannten Indikationen in einer Dosis von 0,1-100 mg per kg Körpergewicht wirksam.

Beispiele für die erfindungsgemäßen Verbindungen sind :

11-[γ-Dimethylamino)-propylthio]-2-äthylsulfonyl-dibenz-[b,f]-thiepin ;
10,11-Dihydro-10-[γ-(N-methylpiperazino)-propylthio]-dibenz-[b,f]-thiepin ;
3-Chlor-11[β-(äthylmethylamino)-äthylthio]-dibenz-[b,f]-thiepin ;
3-Fluor-11-[β-(methylamino)-äthylthio]dibenz-[b,f]-thiepin ;
2-Brom-7-fluoro-11-[β-(dimethylamino)-äthylthio]dibenz-[b,f]-thiepin.

Wirksame Mengen der Verbindungen der Erfindung können auf die unterschiedlichste Art und Weise verabreicht werden, z. B. oral als Kapseln oder Tabletten oder parenteral als sterile Lösungen oder Suspensionen, intravenös als sterile Lösungen. Die erfindungsgemäßen Verbindungen mit freier Base, die allein auch wirksam sind, können aus Gründen der Stabilität, einer besseren kristallisierbarkeit oder einer besseren Löslichkeit etc. in Form ihrer pharmazeutisch verträglichen Additionssalze verabreicht werden.

Die Verbindungen können oral, z. B. mit einem inerten Verdünnungsmittel oder mit einem eßbaren Trägerstoff oder als Gelatinekapseln oder Tabletten verabreicht werden. Zur oralen therapeutischen Verabreichung können die aktiven Verbindungen mit Hilfs- und Trägerstoffen kombiniert in Form von Tabletten, Pastillen, Kapseln, Elixieren, Suspensionen, Sirups, Waffeln, Kaugummi etc. verwendet werden. Der Wirkstoffgehalt sollte in Abhängigkeit von der besonderen Verabreichungsform wenigstens 0,5 %, bezogen auf das Gewicht der Einheitsdosis, vorteilhafterweise 4-70 %, betragen. Er sollte so bemessen sein, daß eine geeignete Dosierung erreicht werden kann. Vorzugsweise sollte eine orale Einheitsdosis aus den Mitteln und Präparaten der vorliegenden Erfindung 1,0-300 mg Wirkstoff enthalten.

Die Tabletten, Pillen, Kapseln, Pastillen etc. können weiterhin Bindemittel wie mikrokristalline Cellulose, Tragantgummi oder Gelatine ; Hilfs- und Trägerstoff wie Stärke oder Laktose, ein Sprengmittel wie Alginsäure, Maisstärke etc. ein Gleitmittel wie Magnesiumstearat oder kolloidales Siliciumdioxid, einen Süßstoff wie Sacharose oder Rohrzucker oder einen Aromastoff wie Pfefferminze, Methylsalizylat

oder Orangearoma enthalten. Im Falle von Kapseln kann die Dosiseinheit zusätzlich eine flüssige Trägersubstanz wie Pflanzenöl enthalten. Andere Dosiseinheiten können Materialien enthalten, die die physikalische Form der Dosiseinheit verändern z. B. Überzüge. So können Tabletten oder Pillen von Zucker, Schellack oder anderen Beschichtungsmitteln überzogen sein. Ein Sirup kann zusätzlich zu den Wirkstoffen Rohrzucker als Süßstoff und bestimmte Konservierungsmittel, Farb- und Aromastoffe enthalten. Die bei der Herstellung dieser verschiedenen Mittel verwendeten Stoffe sollten in den angewandten Mengen pharmazeutisch rein und nicht giftig sein.

Zur parenteralen therapeutischen Verabreichung können die Wirkstoffe gemäß der Erfindung als Lösung oder Suspension vorliegen. Die Präparate sollten wenigstens 0,1 %, im allgemeinen zwischen 0,5-30 %, Wirkstoff, bezogen auf ihr Gewicht enthalten. Der Wirkstoffgehalt in diesen Mitteln sollte so bemessen sein, daß eine entsprechende Dosierung ermöglicht wird. Vorzugsweise werden die Mittel oder Präparate gemäß der vorliegenden Erfindung so hergestellt, daß eine parenterale Dosiseinheit zwischen 0,5-100 mg Wirkstoff enthält.

Die Lösungen oder Suspensionen können noch weitere Komponenten enthalten, nämlich ein steriles Verdünnungsmittel wie Wasser zur Injektion eine Salzlösung, Öle, Polyäthylenglykole, Glyzerin, Propylenglykol oder andere synthetische Lösungsmittel ; antibakterielle Mittel wie Benzylakohol ; Antioxydiermittel wie Ascorbinsäure oder Natriumbisulfit ; Cheliermittel wie Äthylendiamintetraessigsäure ; Puffersubstanzen wie Acetate, Zitrate oder Phosphate und Mittel der Einstellung des osmotischen Drucks. Das parenteral zu verabreichende Präparat kann in Form von Ampullen oder Einwegspritzen aus Glas oder Plastik eingesetzt werden.

Die Erfindung wird in den folgenden Beispielen erläutert :

Beispiel 1

Eine Lösung von 20,0 g 2-Chlor-10,11-Dihydro-11-oxodibenz-[b,f]-thiepin, 21,8 g β-Dimethylaminoäthanthiolhydrochlorid und 218 ml Eisessig wird mit 63 g Bortrifluoridätherat versetzt. Nach beendeter Zugabe wird das Reaktionsgemisch 30 Minuten gerührt und dann 48 Stunden stehen gelassen.

Dann gießt man die Mischung in 1 200 ml einer 10 %igen Natronlauge, worauf die basisch gemachte Mischung mit Methylenchlorid extrahiert wird. Die vereinigten Methylenchloridfraktionen werden mit Wasser gewaschen, getrocknet und filtriert. Dann wird das Filtrat zur Trockene eingedampft, wobei ein rotes Öl zurückbleibt, das an einer Silicagel/Methylenchlorid Kolonne mit einer Mischung aus 2-4 % Methanol in Methylenchlorid als Eluiermittel chromatographiert wird. Das gereinigte Produkt wird in 2-Chlor-11-[β-(dimethylamino)-äthylthio]-dibenz-[b,f]-thiepin-hydrochlorid überführt.

Analyse :
Berechnet für $C_{18}H_{18}ClNS_2 \cdot HCl$ : 56,24 %C ; 4,98 %H ; 3,64 %N.
Gefunden : 56,35 %C ; 5,17 %H ; 3,47 %N.

Beispiel 2

Eine Probe 10,11-Dihydro-10-oxodibenz-[b,f]-thiepin wird gemäß Beispiel 1 zum 10-[β-(Dimethylamino)-äthylthio]-dibenz-[b,f]-thiepin-hydrochlorid umgesetzt.

Beispiel 3

Zu einer Mischung aus 1,08 g β-Dimethylaminoäthanthiolhydrochlorid und 2,5 ml Bortrifluoridätherat wird eine Mischung aus 1,0 g 2-Chlor-10,11-dihydro-11-hydroxy-spirodibenz-[b,f]-thiepin in 8 ml Eisessig zugetropft. Nach beendeter Zugabe wird das Reaktionsgemisch bei Zimmertemperatur 30 Minuten gerührt und dann 24 Stunden stehen gelassen. Dann gibt man die Mischung in 50 ml gesättigter Kaliumcarbonat-Eis Lösung, wie sie mit Äther extrahiert wird. Die vereinigten Ätherextrakte werden nacheinander mit verdünnter Kaliumcarbonatlösung und Wasser gewaschen, getrocknet, filtriert und verdampft, wobei ein Öl zurückbleibt, das an einer Silicagel/Methylenchlorid-Kolonne mit 5 % Methylakohol in Methylenchlorid als Eluiermittel chromatographiert wird und dann in das Maleinsäuresalz, nämlich 2-Chlor-10,11-dihydro-11-[β-(dimethylamino)-äthylthio]-dibenz-[b,f]-thiepinmaleat, überführt wird.

Schmelzpunkt : 99-101 °C.
Analyse :
Berechnet für $C_{18}H_{20}ClNS_2 \cdot C_4H_4O_4$ : 56,70 %C ; 5,19 %H ; 3,01 %N.
Gefunden : 56,70 %C ; 5,29 %H ; 3,03 %N.

Beispiel 4

Eine Probe 10-[β-(dimethylamino)-äthylthio]-dibenz-[b,f]-thiepin (freie Base aus Beispiel 3) wird gemäß Beispiel 3 zum 10,11-Dihydro-10-[β-(dimethylamino)-äthylthio]-dibenz-[b,f]-thiepinhydrochlorid umgesetzt.

Beispiel 5

Eine Lösung aus 1,0 g 2-Chlor-11-[β-(dimethylamino)-äthylthio]-dibenz-[b,f]-thiepin (freie Base aus Beispiel 1) in 10 ml Methylenchlorid wird zu einer gerührten Lösung aus 0,5 g Bromcyan und 1,0 g Kaliumcarbonat in 7 ml Methylenchlorid getropft. Nach beendeter Zugabe läßt man das Reaktionsgemisch stehen, wobei die Reaktion nach ca. 4 Stunden beendet ist. Dann wird das Gemisch filtriert und das Filtrat eingedampft, wobei ein Öl zurückbleibt, das an einer Silicagel/Methylenchlorid-Kolonne mit Methylenchlorid als Eluiermittel chromatographiert wird. Die gewünschte Fraktion wird zur Trockene eingedampft, wobei 2-Chlor-11-[β-(N-Cyano-N-methyl)-aminoäthylthio]-dibenz-[b,f]-thiepin als gelbes Öl anfällt.

Analyse :
Berechnet für $C_{18}H_{15}ClN_2S_2$ : 60,23 %C ; 4,21 %H ; 7,81 %N.
Gefunden : 60,62 %C ; 4,19 %H ; 7,75 %N.

Beispiel 6

Eine Lösung aus 0,95 g 2-Chlor-11-[β-(dimethylamino)-äthylthio]-dibenz-[b,f]-thiepin (freie Base aus Beispiel 1) in 10 ml Methylenchlorid wird zu einer gerührten Lösung aus 0,50 g Chlorameisensäurephenylester und 0,2 g Natriumbicarbonat in 20 ml Methylenchlorid getropft. Nach beendeter Zugabe wird das Reaktionsgemisch nacheinander 24 Stunden bei 25 °C gerührt, filtriert, mit 25 ml Methylenchlorid verdünnt, mit einer 10 %igen Natronlauge und mit Wasser gewaschen, getrocknet und wieder filtriert. Das Filtrat wird dann verdampft, wobei ein gelbes Öl anfällt, das an einer Silicagel/Methylenchlorid Kolonne mit Methylenchlorid als Eluiermittel chromatographiert wird. Nach dem Reinigen erhält man 2-Chlor-11-[β-(N-methyl-N-phenoxycarbonylamino)-äthylthio]-dibenz-[b,f]-thiepin als gelbes Öl.

Analyse :
Berechnet für $C_{24}H_{20}ClNO_2S_2$ : 63,49 %C ; 4,44 %H ; 3,09 %N.
Gefunden : 63,76 %C ; 4,49 %H ; 2,90 %N.

In ähnlicher Weise kann eine kleine Probe 10-[β-(dimethylamino)-äthylthio]-dibenz-[b,f]-thiepin (freie Base aus Beispiel 2) zum 10-[β-(N-Methyl-N-phenoxycarbonylamino)-äthylthio]-dibenz-[b,f]-thiepin umgesetzt werden.

Beispiel 7

Eine Lösung aus 5,6 g 2-Chlor-11-[β-(N-methyl-N-phenoxycarbonylamino)-äthylthio]-dibenz-[b,f]-thiepin (Beispiel 6), 127 ml Äthylenglykol und 10,8 Kaliumhydroxyd wird 30 Minuten bei 150-155 °C gerührt. Dann gibt man die Reaktionsmischung in 300 ml Eiswasser und extrahiert die wäßrige Mischung mit einer Äther-Toluol-Mischung (1 : 1). Die vereinigten Extrakte werden nacheinander sorgfältig mit Wasser gewaschen, getrocknet und filtriert, und das Filtrat wird zu einem organgefarbenen Öl eingedampft. Dieses Öl wird nun in sein HCl-Additionssalz überführt, das aus einer Methanol-Aceton-Äther-Mischung zum 2-Chlor-11-[β-(methylamino)-äthylthio]-dibenz-[b,f]-thiepin-hydrochlorid umkristallisiert wird. Schmelzpunkt : 194-196 °C.

Analyse :
Berechnet für $C_{17}H_{16}ClNS_2 \cdot HCl$ : 55,13 %C ; 4,63 %H ; 3,78 %N.
Gefunden : 55,28 %C ; 4,71 %H ; 3,93 %N.

Verfährt man wie oben beschrieben unter Verwendung des oben erwähnten 10-[β-(N-methyl-N-phenoxycarbonylamino)-äthylthio]-dibenz-[b,f]-thiepin, so erhält man das 10-[β-(methylamino)-äthylthio]-dibenz-[b,f]-thiepin-hydrochlorid.

Beispiel 8

Eine Lösung aus 2-Chlor-10,11-dihydro-11-[β-(Dimethylamino)-äthylthio]-dibenz-[b,f]-thiepin (freie Base aus Beispiel 3) in 10 ml Chloroform wird zu einer Lösung aus einer stöchiometrischen Menge Bromcyan und einem Überschuß an Kaliumcarbonat in 5 ml Chloroform getropft. Nach beendeter Zugabe läßt man die Reaktionsmischung 10 Minuten stehen und filtriert sie dann. Das Filtrat wird zum 11-(β-Bromäthylthio)-2-Chlor-10,11-dihydrodibenz-[b,f]-thiepin eingedampft.

Beispiel 9

Eine Mischung aus stöchiometrischen Mengen von 2-Chlor-11-(β-bromäthylthio)-10,11-dihydrodibenz-[b,f]-thiepin aus Beispiel 8 und N-methylpiperazin, überschüssigem Natriumbicarbonat und 1,0 g

Kaliumiodid in 15 ml DMF wird 16 Stunden bei 80 °C gerührt. Man läßt die Mischung abkühlen und verdünnt sie dann mit Wasser. Das Zweiphasengemisch wird dreimal mit je 100 ml Äther extrahiert, die Ätherextrakte werden vereinigt und heftig mit einem großen Überschuß an 2 n-Salzsäure geschüttelt. Die saure Lösung wird basisch gemacht, wodurch das freie Amin frei gesetzt wird, welches dann mit Benzol extrahiert wird. Die benzolische Lösung wird getrocknet. Dann wird das Benzol im Vakuum entfernt, wobei 2-Chlor-10,11-dihydro-11-[β-(4-Methylpiperazinyl-1-yl)-äthylthio]-dibenz-[b,f]-thiepin zurückbleibt.

Verfährt man wie oben, jedoch unter Verwendung von Morpholin anstelle von N-Methylpiperazin, so erhält man 2-Chlor-10,11-dihydro-11-(β-morpholinoäthylthio)-dibenz-[b,f]-thiepin.

Beispiele 10 und 11

Verfährt man wie in Beispiel 2 und ersetzt β-Dimethylaminoäthanthio-hydrochlorid durch β-Diisopropylaminoäthanthiol-hydrochlorid bzw. β-Diäthylaminoäthanthiolhydrochlorid, so erhält man in Beispiel 10 10-[β-(Diisopropylamino)-äthylthio]-dibenz-[b,f]-thiepin-hydrochlorid und in Beispiel 11 10-[β-(Diäthylamino)-äthylthio]-dibenz-[b,f]-thiepin-hydrochlorid.

Beispiel 12

a) Eine Lösung aus stöchiometrischen Mengen von 2-(4-Methylsulfonylphenylthio)-benzylnitril und 85 %igem Kaliumhydroxid in einem Alkohol-Wasser-Gemisch wird 24 Stunden bei 115 °C gerührt. Dann dampft man das Reaktionsgemisch ein, wobei ein Öl zurückbleibt, das in Wasser gelöst wird. Die erhaltene wäßrige Lösung wird nacheinander mit Äther gewaschen und mit verdünnter Salzsäure angesäuert, wobei sich wiederum ein Öl bildet, das in Methylenchlorid gelöst wird. Die Methylenchloridlösung wird getrocknet, filtriert und zur Trockene eingedampft, wobei man 2-(4-Methylsulfonylphenylthio)-phenylessigsäure erhält.

b) Eine Mischung von 1,0 g 2-(4-Methylsulfonylphenylthio)-phenylessigsäure und 10 ml Polyphosphorsäure wird unter Stickstoff 2 Stunden bei 90-100 °C gerührt. Dann läßt man das Reaktionsgemisch abkühlen und gießt es in 100 ml eines Eiswasserbreies. Die wäßrige Lösung wird mit 20 %iger Natronlauge basisch gemacht und dann mit Methylenchlorid extrahiert. Die vereinigten Extrakte werden getrocknet und zur Trockene eingedampft, wobei man ein Öl erhält, das an einer Silicagel-Kolonne mit 2 %igem Methanol in Methylenchlorid als Eluiermittel chromatographiert wird. Die chromatographierte Lösung wird zur Trockene eingedampft, wobei 10,11-Dihydro-2-methylsulfonyl-11-oxodibenz-[b,f]-thiepin zurückbleibt.

c) Eine Lösung von stöchiometrischen Mengen von 10,11-Dihydro-2-methylsulfonyl-11-oxodibenz-[b,f]-thiepin und Dimethylaminoäthanthiol-hydrochlorid mit 15 ml Bortrifluorid-ätherat in 37 ml Eisessig wird gerührt, bis die Reaktion beendet ist. Dann gibt man das Reaktionsgemisch in 300 ml kalte Natronlauge und extrahiert die erhaltene Lösung mit Methylenchlorid. Die vereinigten Extrakte werden mit Wasser gewaschen, getrocknet und filtriert. Das Filtrat wird zur Trockene eingedampft, wobei ein Öl zurückbleibt, das an einer Silicagel-Kolonne mit 2-4 % Methanol in Methylenchlorid als Eluiermittel chromatographiert wird. Die chromatographierte Lösung wird zur Trockene eingedampft, wobei man 11-[(β-Dimethylamino)-äthylthio]-2-methylsulfonyl-dibenz-[b,f]-thiepin erhält.

**Ansprüche**

1. Eine Verbindung der Formel

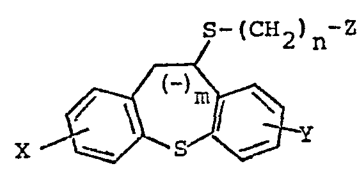

worin

X und Y gleich oder verschieden sein können und jeweils Wasserstoff, Halogen, $C_{1-6}$-Alkylsulfonyl bedeuten,

Z für die Gruppe

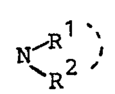

steht, wobei $R^1$ Wasserstoff,
geradkettiges oder verzweigtes $C_1$-$C_6$-Alkyl oder Cyano bedeutet und $R^2$ geradkettig oder verzweigtes $C_{1-6}$-Alkyl bedeutet, oder $R^1$ und $R^2$ zusammen mit dem Stickstoffatom, an das sie gebunden sind, den Morpholin- oder einen N-substituierten Piperazin-Ring, worin der Substituent $C_{1-6}$-Alkyl ist, bilden,

m für 0 oder 1 steht und

n eine Zahl von 2 bis 4 bedeutet,

sowie deren physiologisch verträgliche Säureadditionssalze.

2. 2-Chloro-11-[β-(dimethylamino)-äthylthio]-dibenz-[b,f]-thiepin und seine physiologisch verträglichen Säureadditionssalze.

3. 10-[β-(Dimethylamino)-äthylthio]-dibenz-[b,f]-thiepin und seine physiologisch verträglichen Säureadditionssalze.

4. 2-Chlor-10,11-dihydro-11-[β-(dimethylamino)-äthylthio]-dibenz-[b,f]-thiepin und seine physiologisch verträglichen Säureadditionsalze.

5. 10,11-Dihydro-10-[β-(dimethylamino)-äthylthio]-dibenz-[b,f]-thiepin und seine physiologisch verträglichen Säureadditionssalze.

6. 11-(β-Bromäthylthio)-2-chlor-10,11-dihydrobenz-[b,f]-thiepin und seine physiologisch verträglichen Säureadditionssalze.

7. 11-[β-Dimethylamino)-äthylthio-2-methylsulfonyl]-dibenz-[b,f]-thiepin und seine physiologisch verträglichen Säureadditionssalze.

8. 10-[β-(Methylamino)-äthylthio]-dibenz-[b,f]-thiepin und seine physiologisch verträglichen Säureadditionssalze.

9. 2-Chlor-11-[β-(methylamino)-äthylthio]-dibenz-[b,f]-thiepin und seine physiologisch verträglichen Säureadditionssalze.

10. Eine Verbindung gemäß Anspruch 1 zur Verwendung als Arzneimittel, mit antidepressiver, analgetischer und anticonvulsiver Wirkung.

11. Verfahren zur Herstellung von Verbindungen der Formel I

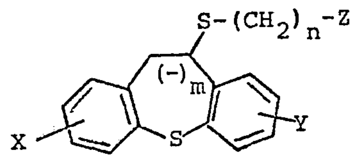

(I)

worin

X und Y gleich oder verschieden sein können und jeweils Wasserstoff, Halogen, $C_{1-6}$-Alkylsulfonyl bedeuten,

Z für ein Halogenatom oder die Gruppe

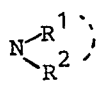

steht, wobei $R^1$ Wasserstoff, geradkettiges oder verzweigtes $C_1$-$C_6$-Alkyl, Phenoxycarbonyl, $C_{1-6}$-Alkoxycarbonyl oder falls $R^2$ Methyl ist, Cyano bedeutet und $R^2$ geradkettig oder verzweigtes $C_{1-6}$-Alkyl bedeutet, oder $R^1$ und $R^2$ zusammen mit dem Stickstoffatom, an das sie gebunden sind, den Morpholin- oder einen N-substituierten Piperazin-Ring, worin der Substituent $C_{1-6}$-Alkyl ist, bilden,

m für 0 oder 1 steht und

n eine Zahl von 2 bis 4 bedeutet,

sowie deren physiologisch verträgliche Säureadditionssalze, dadurch gekennzeichnet, daß man

a) eine Verbindung der Formel

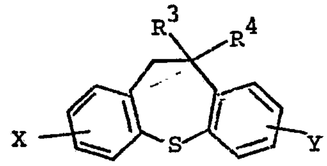

(II)

worin X und Y die in Formel I angegebene Bedeutung haben und $R^3$ und $R^4$ verschieden sind und Wasserstoff oder Hydroxy oder zusammen Sauerstoff bedeuten, mit Aminoalkylthiol der Formel

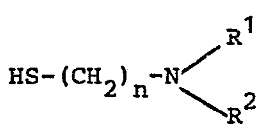

(III)

worin $R^1$ und $R^2$ gleich oder verschieden sind und geradkettiges oder verzweigtes $C_{1-6}$-Alkyl bedeuten, in Gegenwart eines Katalysators/Dehydratisierungsmittels zur Verbindung der Formel

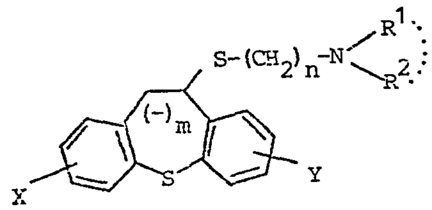

(Ia)

umsetzt und

b) gegebenenfalls eine Verbindung der Formel Ia, worin $R^1$ und $R^2$ Methyl bedeuten, mit Halogencyan zu einem Gemisch einer Verbindung der Formel

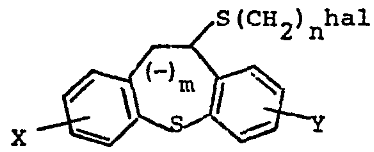

(Ib)

und einer Verbindung der Formel

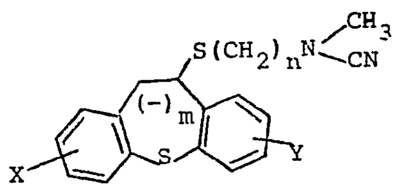

(Ic)

worin X, Y, m und n die in Formel I gegebene Bedeutung haben und Hal Halogen bedeutet, umsetzt und die beiden Verbindungen isoliert,

c) gegebenenfalls eine Verbindung der Formel Ib in bekannter Weise mit einem geeigneten nicht zyklischen oder zyklischen Amin zur entsprechenden Verbindung der Formel

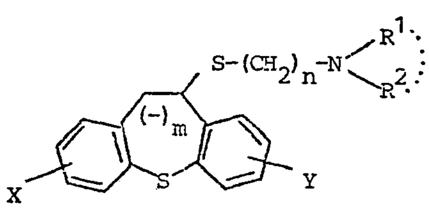

(Ia)

worin $R^1$ Wasserstoff, verzweigtes oder unverzweigtes $C_{1-6}$-Alkyl, $R_2$ geradkettiges oder verzweigtes $C_{1-6}$-Alkyl oder $R^1$ und $R^2$ zusammen mit dem Stickstoffatom, an das sie gebunden sind, den Morpholin- oder einen am N-substituierten Piperazin-Ring, wobei der Substituent $C_{1-6}$-Alkyl ist, bilden,

d) gegebenenfalls eine Verbindung der Formel Ia, worin $R^1$ und $R^2$ gleich oder verschieden sein können und geradkettiges oder verzweigtes $C_{1-6}$-Alkyl bedeuten und X, Y, m und n die in Formel I angegebene Bedeutung haben, mit einem $C_{1-6}$-Alkyl- oder Phenyl-Chlorameisensäureester zu einer Verbindung der Formel Ia, worin $R^1$ $C_{1-6}$-Alkoxy oder Phenoxycarbonyl bedeutet und $R^2$, X, Y, m und n die in Formel I angegebene Bedeutung haben, umsetzt und

e) eine Verbindung der Formel Ia, worin X, Y, m und n die in Formel I angegebene Bedeutung haben, $R^1$ $C_{1-6}$-Alkoxy oder Phenoxycarbonyl bedeutet und $R^2$ für geradkettiges oder verzweigtes $C_{1-6}$-Alkyl steht, in einem alkalischen oder sauren Medium zur Verbindung der Formel Ia, worin X, Y, m und n die Formel I angegebene Bedeutung haben, $R^2$ die oben angegebene Bedeutung hat und $R^1$ für Wasserstoff steht, umsetzt und

f) gegebenenfalls eine Verbindung der Formel Ia, worin X, Y, m und n die in Formel I angegebene Bedeutung haben $R^1$ Wasserstoff und $R^2$ geradkettiges oder verzweigtes $C_{1-6}$-Alkyl bedeutet, mit einem geradkettigen oder verzweigten $C_{1-6}$-Alkylhalogenid, zu einer Verbindung der Formel Ia, worin X, Y, m und n die in Formel I angegebene Bedeutung haben, $R^1$ geradkettiges oder verzweigtes $C_{1-6}$-Alkyl, bedeutet und $R^2$ für geradkettiges oder verzweigtes $C_{1-6}$-Alkyl steht, umsetzt.

12. Verbindungen der Formel

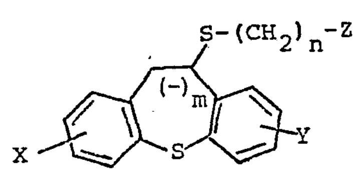

(Ia,b)

worin X, Y, m und n die zur Formel I in Anspruch 1 genannten Bedeutungen haben, Z ein Halogenatom oder den Rest

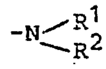

steht, wobei $R^1$ ein Phenoxycarbonyl oder $C_{1-6}$-Alkoxycarbonyl und $R^2$ geradkettiges oder verzweigtes $C_{1-6}$-Alkyl bedeuten, zur Herstellung der Verbindungen nach Anspruch 1.

## Claims

1. A compound of the formula

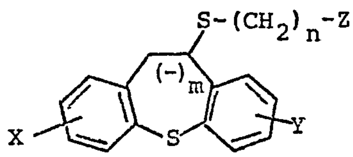

wherein

X and Y are the same or different and each can be hydrogen, halogen $C_1$-$C_6$-alkylsulfonyl,

Z is

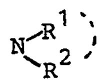 ;

wherein $R^1$ is hydrogen, straight or branched chain $C_1$-$C_6$-alkyl or, in case $R^2$ is methyl, cyano $R^2$ is straight or branched chain $C_1$-$C_6$-alkyl ; and when $R^1$ and $R^2$ are taken together with the nitrogen atoms to which they are attached, form a morpholino, or N-substituted piperazinyl in which the substituent is $C_1$-$C_6$-alkyl ;

m is the integer 0 or 1 ; and

n is an integer of from 2 to 4 ;

and its physiologically tolerable acid addition salts.

2. 2-Chloro-11-[β-(dimethylamino)-ethylthio]-dibenz-[b,f]-thiepin and its physiologically tolerable acid addition salts.

3. 10-[β-(Dimethylamino)-ethyithio]-dibenz-[b,f]-thiepin and its physiologically tolerable acid addition salts.

4. 2-Chloro-10,11-dihydro-11-[β-(dimethylamino)-ethylthio]-dibenz-[b,f]-thiepin and its physiologically tolerable acid addition salts.

5. 10,11-Dihydro-10-[β-(dimethylamino)-ethylthio]-dibenz-[b,f]-thiepin and its physiologically tolerable acid addition salts.

6. 11-(β-Bromoethylthio)-2-chloro-10,11-dihydrobenz-[b,f]-thiepin and its physiologically tolerable acid addition salts.

7. 11-[(β-dimethylamino)-ethylthio-2-methylsulfonyl]-dibenz-[b,f]-thiepin and its physiologically tolerable acid addition salts.

8. 10-[β-(methylamino)-ethylthio]-dibenz-[b,f]-thiepin and its physiologically tolerable acid addition salts.

9. 2-Chloro-11-[β-(methylamino)-ethylthio]-dibenz-[b,f]-thiepin and its physiologically tolerable acid addition salts.

10. A compound as defined in claim 1 for use as medicament with antidepressive, analgetic and anticonvulsant activity.

11. A process for preparing compounds of the formula I

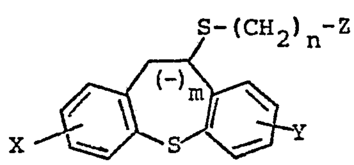

(I)

wherein

X and Y are the same or different and each can be hydrogen, halogen or $C_1$-$C_6$-alkylsulfonyl ;

Z is halogen or

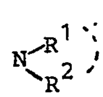

wherein $R^1$ is hydrogen, straight or branched chain $C_1$-$C_6$ alkyl, phenoxycarbonyl, $C_1$-$C_6$-alkoxycarbonyl, or, in case $R^2$ is methyl, cyano ; $R^2$ is straight or branched chain $C_1$-$C_6$-alkyl or, when $R^1$ and $R^2$ are taken together with the nitrogen atom to which they are attached, form a morpholino, or N-substituted piperazinyl in which the substituent is $C_1$-$C_6$-alkyl ;

m is the integer 0 or 1 ; and

n is an integer of from 2 to 4 ;

and its physiologically tolerable acid addition salts, which comprises

a) reacting a compound of the formula

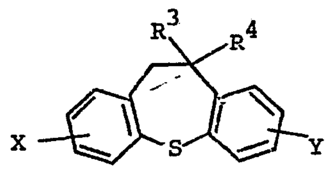

(II)

wherein X and Y are as defined in formula I and $R^3$ and $R^4$ are different and each is hydrogen or hydroxy, or $R^3$ and $R^4$ together are oxygen, with aminoalkylthiol of the formula

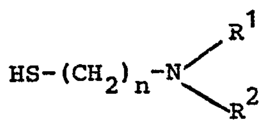

(III)

wherein $R^1$ and $R^2$ are the same or different and each can be a straight or branched chain $C_1$-$C_6$-alkyl in the presence of a catalyst/dehydrating agent to produce a compound of the formula

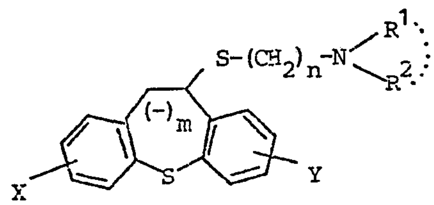

(Ia)

and

b) optionally reacting a compound of the formula Ia wherein $R^1$ and $R^2$ are each methyl with cyanogen halide to obtain a mixture of one compound of the formula

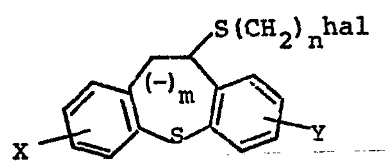

(Ib)

and another compound of the formula

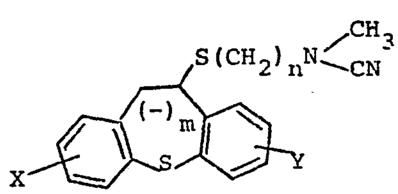

(Ic)

wherein X, Y, m and n are as defined in formula I and hal is halogen, and isolating each of the two compounds, and

c) optionally reacting a compound of the formula Ib in a known fashion with an appropriate noncyclic or cyclic amine to obtain the corresponding compound of the formula

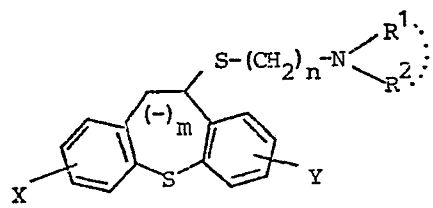

(Ia)

wherein $R^1$ is hydrogen, straight or branched chain $C_1$-$C_6$-alkyl ; or $R^2$ is straight or branched chain $C_1$-$C_6$ alkyl ; or when $R^1$ and $R^2$ are taken together with the nitrogen atom to which they are attached, form a morpholino or N-substituted piperazinyl in which the substituent is $C_1$-$C_6$-alkyl.

d) optionally reacting a compound of the formula Ia, wherein $R^1$ and $R^2$ are the same or different and each is a straight or branched chain $C_1$-$C_6$-alkyl, and X, Y, m, n are as defined in formula I with an $C_1$-$C_6$-alkyl or phenyl chloroformate to obtain a compound of the formula Ia wherein $R^1$ is $C_1$-$C_6$-alkoxy or phenoxy carbonyl and $R^2$, X, Y, m and n are as defined in formula I, and

e) reacting a compound of the formula Ia, wherein X, Y, m and n are as defined in formula I, $R^1$ is $C_1$-$C_6$-alkoxy or phenoxy carbonyl and $R^2$ is a straight or branched chain $C_1$-$C_6$-alkyl, with a base or an acid medium to obtain a compound of the formula Ia wherein X, Y, m and n are as defined in formula I, $R^2$ is as defined above and $R^1$ is hydrogen, and

f) optionally reacting a compound of the formula Ia, wherein X, Y, m and n are as defined in formula I, $R^1$ is hydrogen and $R^2$ is a straight or branched chain $C_1$-$C_6$-alkyl with a straight or branched chain $C_1$-$C_6$-alkyl halide, to obtain a compound of the formula Ia in which X, Y, m and n are as defined in formula I, $R^1$ is straight or branched chain $C_1$-$C_6$ alkyl, and $R^2$ is straight or branched chain $C_1$-$C_6$-alkyl.

12. A compound of the formula

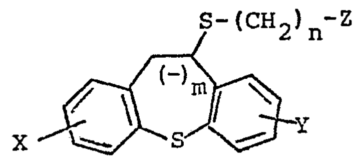

(Ia,b)

wherein X, Y, m and n are as defined in formula I of claim 1, Z is halogen or

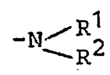

wherein $R^1$ is phenoxycarbonyl or $C_1$-$C_6$-Alkoxycarbonyl and $R^2$ is straight or branched $C_1$-$C_6$-Alkyl for the use in the preparation of compounds as defined in claim 1.

**Revendications**

1. Un composé de formule

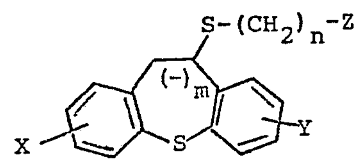

dans laquelle

X et Y peuvent être identiques ou différents et représentent chacun un atome d'hydrogène, un atome d'halogène, un groupe alcoyl sulfonyle dans lequel la partie alcoyle est en $C_{1\text{-}6}$,

Z représente le groupe

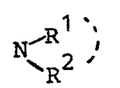

où $R^1$ est un atome d'hydrogène,
un groupe alcoyle en $C_1$-$C_6$ à chaîne droite ou ramifiée ou si $R^2$ est un groupe méthyle, un groupe cyano, et $R^2$ est un groupe alcoyle en $C_{1\text{-}6}$ à chaîne droite ou ramifiée, ou $R^1$ et $R^2$ forment ensemble avec l'atome d'azote sur lequel ils sont fixés, le noyau morpholine ou un noyau pipérazine N-substituée, où le substituant est un groupe alcoyle en $C_{1\text{-}6}$,

m est 0 ou 1, et

n est un nombre de 2 à 4,

ainsi que ses sels d'addition avec des acides physiologiquement acceptables.

2. 2-Chloro-11-[β-(diméthylamino)-éthylthio]-dibenzo-[b,f]-thiépine et ses sels d'addition avec des acides physiologiquement acceptables.

3. 10-[β-(Diméthylamino) éthylthio]-dibenzo-[b,f]-thiépine et ses sels d'addition avec des acides physiologiquement acceptables.

4. 2-Chloro-10,11-dihydro-11-[β-(diméthylamino)-éthylthio]-dibenzo[b,f]-thiépine et ses sels d'addition avec des acides physiologiquement acceptables.

5. 10,11-Dihydro-10[β-(diméthylamino)-éthylthio]-dibenzo[b,f]-thiépine et ses sels d'addition avec des acides physiologiquement acceptables.

6. 11-(β-Bromoéthylthio)-2-chloro-10,11-dihydrobenzo-[b,f]-thiépine et ses sels d'addition avec des acides physiologiquement acceptables.

7. 11-[β-Diméthylamino)-éthylthio-2-méthylsulfonyl]-dibenzo[b,f]-thiépine et ses sels d'addition avec des acides physiologiquement acceptables.

8. 10-[β-(Méthylamino)-éthylthio]-dibenzo-[b,f]-thiépine et ses sels d'addition avec des acides physiologiquement acceptables.

9. 2-Chloro-11-[β-(méthylamino)-éthylthio]-dibenzo-[b,f]-thiépine et ses sels d'addition avec des acides physiologiquement acceptables.

10. Un composé selon la revendication 1, destiné à être utilisé comme médicament, avec une action antidépressive, analgésique et anticonvulsive.

11. Procédé pour la préparation de composés de formule I

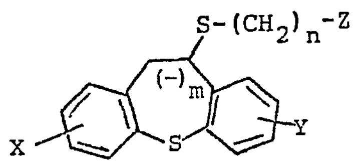

(I)

dans laquelle

X et Y peuvent être identiques ou différents et représentent chacun un atome d'hydrogène, un atome d'halogène, un groupe alcoyl-sulfonyle dans lequel la partie alcoyle est en $C_{1-6}$,

Z représente un atome d'halogène ou le groupe

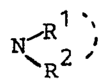

où $R^1$ est un atome d'hydrogène, un groupe alcoyle en $C_{1-6}$ à chaîne droite ou ramifiée, phénoxycarbonyle, alcoxycarbonyle en $C_{1-6}$ ou, si $R^2$ est un groupe méthyle, un groupe cyano, et $R^2$ est un groupe alcoyle en $C_{1-6}$ à chaîne droite ou ramifiée, ou $R^1$ et $R^2$ ensemble forment avec l'atome d'azote sur lequel ils sont fixés, le noyau morpholine ou un noyau pipérazine N-substituée, où le substituant est un groupe alcoyle en $C_{1-6}$,

m est 0 ou 1 et

n est un nombre de 2 à 4,

ainsi que leurs sels d'addition avec des acides physiologiquement acceptables, lequel procédé est caractérisé en ce que

a) on fait réagir un composé de formule

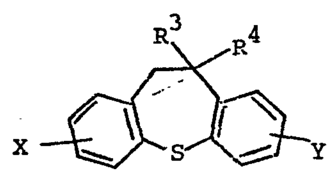

(II)

dans laquelle X et Y ont les significations indiquées dans la formule I et $R^3$ et $R^4$ sont différents et représentent un atome d'hydrogène ou un groupe hydroxy ou représentent ensemble un atome d'oxygène, avec un aminoalcoylthiol de formule

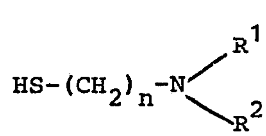

(III)

dans laquelle $R^1$ et $R^2$ sont identiques ou différents et représentent un groupe alcoyle en $C_{1-6}$ à chaîne droite ou ramifiée, en présence d'un catalyseur/agent de déshydratation pour obtenir un composé de formule

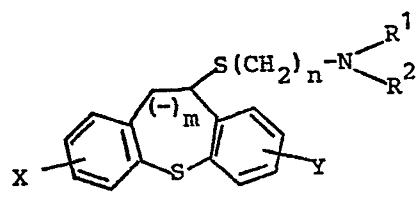

(Ia)

et

b) éventuellement on fait réagir un composé de formule Ia où $R^1$ et $R^2$ représentent un groupe méthyle, avec un halogénure de cyanogène pour obtenir un mélange d'un composé de formule

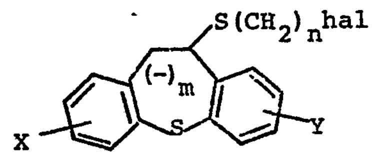

(Ib)

et d'un composé de formule

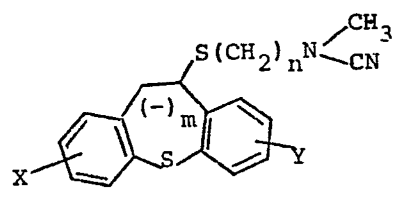

(Ic)

dans lesquelles X, Y, m et n ont les significations indiquées dans la formule I et Hal désigne un atome d'halogène, et l'on isole les deux composés,

c) éventuellement on fait réagir un composé de formule Ib d'une façon connue avec une amine non cyclique ou cyclique correspondante pour obtenir un composé correspondant de formule

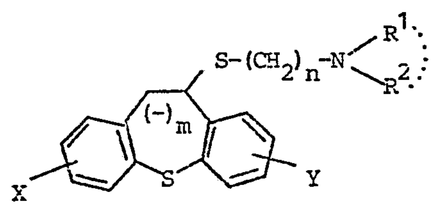

(Ia)

dans laquelle $R^1$ représente un atome d'hydrogène ou un groupe alcoyle en $C_{1-6}$ à chaîne droite ou ramifiée, $R^2$ représente un groupe alcoyle en $C_{1-6}$ à chaîne droite ou ramifiée ou $R^1$ et $R^2$ ensemble forment avec l'atome d'azote sur lequel ils sont fixés, le noyau morpholine ou un noyau pipérazine N-substituée où le substituant est un groupe alcoyle en $C_{1-6}$,

d) éventuellement on fait réagir un composé de formule Ia, où $R^1$ et $R^2$ peuvent être identiques ou différents et représentent un groupe alcoyle en $C_{1-6}$ à chaîne droite ou ramifiée et X, Y, m et n ont les significations indiquées dans la formule I, avec un ester d'acide alcoyl($C_{1-6}$)- ou phénylchloroformique pour obtenir un composé de formule Ia dans laquelle $R^1$ représente un groupe alcoxy($C_{1-6}$)- ou phénoxycarbonyle et $R^2$, X, Y, m et n ont les significations indiquées dans la formule I,

e) on fait réagir un composé de formule Ia, où X, Y, m et n ont les significations indiquées dans la formule I, $R^1$ représente un groupe alcoxy($C_{1-6}$)- ou phénoxycarbonyle et $R^2$ représente un groupe alcoyle en $C_{1-6}$ à chaîne droite ou ramifiée, dans un milieu alcalin ou acide, pour obtenir un composé de formule Ia, où X, Y, m et n ont les significations indiquées pour la formule I, $R^2$ a la signification indiquée ci-dessus et $R^1$ représente un atome d'hydrogène, et

f) éventuellement on fait réagir un composé de formule Ia, où X, Y, m et n ont les significations indiquées dans la formule I, $R^1$ représente un atome d'hydrogène et $R^2$ représente un groupe alcoyle en $C_{1-6}$ à chaîne droite ou ramifiée, avec un halogénure d'alcoyle en $C_{1-6}$ à chaîne droite ou ramifiée pour obtenir un composé de formule Ia, où X, Y, m et n ont les significations indiquées dans la formule I, $R_1$ représente un groupe alcoyle en $C_{1-6}$ à chaîne droite ou ramifiée et $R^2$ représente un groupe alcoyle en $C_{1-6}$ à chaîne droite ou ramifiée.

12. Composé de formule

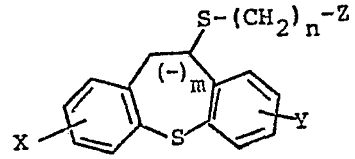

(Ia,b)

dans laquelle X, Y, m et n ont les significations indiquées pour la formule I selon la revendication 1, Z est un atome d'halogène ou le groupe

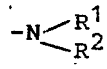
,

où $R^1$ représente un groupe phénoxycarbonyle ou alcoxy $(C_{1-6})$-carbonyle et $R^2$ représente un groupe alcoyle en $C_1$-$C_6$ à chaîne droite ou ramifiée, pour la préparation de composés selon la revendication 1.